(19) Europäisches Patentamt
European Patent Office
Office européen des brevets

(11) Numéro de publication : **0 193 692**
**B1**

(12) **FASCICULE DE BREVET EUROPÉEN**

(45) Date de publication du fascicule du brevet : **05.04.89**

(51) Int. Cl.⁴ : **A 61 K 7/06**, G 01 N 33/48

(21) Numéro de dépôt : **85402633.3**

(22) Date de dépôt : **26.12.85**

(54) **Procédé pour évaluer l'état de surface des fibres kératiniques et composition pour la mise en oeuvre de ce procédé.**

(30) Priorité : **16.01.85 FR 8500566**

(43) Date de publication de la demande : **10.09.86 Bulletin 86/37**

(45) Mention de la délivrance du brevet : **05.04.89 Bulletin 89/14**

(84) Etats contractants désignés : **AT BE CH DE GB IT LI NL**

(56) Documents cités :
**FR-A- 1 101 904**
**GB-A- 2 027 195**

(73) Titulaire : **L'OREAL**
**14, Rue Royale**
**F-75008 Paris (FR)**

(72) Inventeur : **Arnaud, Jean-Claude**
**35, rue Vauvenargues**
**F-75018 Paris (FR)**
Inventeur : **Bore, Pierre**
**197, avenue Daniel Perdrigé**
**F-93370 Montfermeil (FR)**

(74) Mandataire : **Peuscet, Jacques et al**
**Cabinet Peuscet 68, rue d'Hauteville**
**F-75010 Paris (FR)**

**EP 0 193 692 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé pour évaluer l'état de surface des fibres kératiniques, notamment des cheveux humains et des poils. Elle a également pour objet une composition pour la mise en œuvre de ce procédé.

Les spécialistes des fibres kératiniques, qu'il s'agisse des chercheurs ou des praticiens, comme les coiffeurs ou les perruquiers, ont souvent besoin d'évaluer rapidement la qualité de la fibre kératinique sur laquelle ils travaillent. Cette évaluation est généralement effectuée par le spécialiste dans l'optique de savoir comment un traitement chimique ou cosmétique, de type réduction, oxydation, décoloration, permanente, coloration, etc... sera reçu et supporté par le cheveu ou le poil.

Certes, en se basant sur l'expérience, le praticien peut juger de l'état de surface des fibres kératiniques d'après leur aspect et leur toucher. Toutefois, cette appréciation n'est pas rigoureuse, car elle est souvent partielle, parfois subjective, et généralement non chiffrée. On peut donc difficilement se baser sur une telle méthode pour comparer entre eux les états de surface des différentes fibres kératiniques.

L'état de la technique connu concerne un domaine voisin mais différent, à savoir celui de l'industrie de la laine. On peut, en effet, citer un certain nombre de méthodes, qui sont décrites dans la littérature et qui sont destinées à évaluer la qualité de la laine après des traitements industriels.

En premier lieu, on peut mentionner des tests, qui mettent en évidence les dégradations dues à la chlorination et aux traitements mécaniques ou alcalins. Ce sont, notamment le test de coloration au rouge Kiton G, décrit par Carter et Consden dans « J. Text. Inst. (1946) 37, T 227 », le test de coloration au bleu de méthylène, décrit par Whewell et Austerlitz dans « J. Soc. Dy. Col (1943) 59, 45 », le test de coloration au carmin indigo, décrit par Grieve dans « J. Text. Inst. (1946) 37, T 267 », le test d'absorption d'orange II, décrit par Mac Laren dans « Arch. Biochem. Biophys. (1960) 80 », et le test au Lacto bleu (bleu coton IV en solution acide lactique-phénol), décrit par Robinet et Bielen dans « Ann. Sec. Text. Belge (1956) 4-12, 31 ».

En second lieu, on peut mentionner également des tests, qui mettent en évidence des ruptures de liaisons peptidiques et de liaisons disulfure. Ce sont, notamment, le test de solubilité alcaline décrit par Harris et Smith dans « Amer. Dyest. Rep. (1936) 25, 542 », le test de solubilité dans le phénol-acide thioglycolique, décrit par Speakman et Menkart dans « Bull. Inst. Text. Fr. (1952) 30, 315 » et le test de solubilité dans l'urée-bisulfite, décrit par Lees et Elsworth dans « J. Soc. Dy. Col. (1954) 70, 354 ».

Tous ces tests demandent plusieurs heures, voire plus d'une journée, pour apporter une réponse souvent très partielle et d'un intérêt limité pour le coiffeur.

On connaît également, toujours pour évaluer la qualité de la laine ayant subi des traitements industriels, des méthodes d'étude de la porosité, à savoir l'adsorption de paranitrophénol, décrite par Jovanovic dans « App. Pol. Symp. (1971) 915 » et l'adsorption de dextranes, décrite par Schutz dans « S. Int. Wool. Text. Res. Conf. (1975) Vol. II, 446 ».

Ces méthodes d'étude de la porosité mettent en œuvre des techniques complexes et nécessitent un appareillage coûteux. Elles ne sont pas non plus généralisables dans les salons de coiffure.

On sait, par ailleurs, que la fibre kératinique réagit vis-à-vis des solutions de traitement, à la fois, par l'excédent de charges ioniques présent à la surface de ces fibres, et par la porosité de cette surface due à la sensibilisation de la fibre.

On entend ici par « porosité » l'ensemble des phénomènes physiques de surface dont la commune caractéristique est de permettre un libre passage des molécules de réactif à travers les couches cuticulaires des fibres kératiniques.

Ainsi, par exemple, le document GB-A-20 277 195 concerne un procédé pour évaluer l'état ionique des cheveux en utilisant des dérivés du nitrobenzène en milieu acide. Ces colorants servent d'adjuvants ou de nuanceurs de coloration qui, dans les conditions choisies, sont incapables de changer leur couleur en fonction du pH et leur intensité lorsqu'ils ne réagissent pas avec les cheveux.

La société déposante a découvert qu'il existe une corrélation étroite entre, d'une part, les cinétiques d'échange entre les molécules non chargées et les fibres kératiniques, et d'autre part, la porosité en surface des fibres, autrement dit de leur degré de sensibilisation en surface. A partir de cette constatation, elle a mis au point un procédé pour évaluer la qualité de surface des fibres kératiniques, consistant à faire « monter » à travers les couches superficielles desdites fibres des molécules non chargées, colorées ou dont la coloration peut être facilement révélée, par immersion des fibres à étudier dans une solution contenant lesdites molécules, puis à extraire en solution les molécules, qui étaient « montées » sur les fibres kératiniques, la quantité de molécules contenues dans la solution d'extraction étant fonction de la porosité, et par conséquent, de l'état de surfaces des fibres kératiniques.

La molécule utilisée doit remplir les conditions suivantes :

— en premier lieu, la molécule doit, en dehors des conditions précitées, présenter une taille lui permettant de pénétrer dans les interstices des couches superficielles des fibres kératiniques ;

— en deuxième lieu, la molécule doit être généralement soluble en milieu aqueux pour permettre la réalisation de solutions présentant une coloration, naturelle ou après révélation, avant une intensité suffisante ;

— en troisième lieu, la molécule doit être stable dans les conditions de mise en œuvre du procédé ;

— en quatrième lieu, la molécule ne doit comporter aucune charge ionique dans les conditions de mise en œuvre du procédé, afin de ne pas interférer avec la mesure de l'état ionique du cheveu.

Par ailleurs, le principe qui est à la base de l'invention étant que la cinétique de montée de la molécule uniquement sur les couches superficielles des fibres kératiniques est corrélée à l'état de surface de ces fibres, la durée de l'opération, qui consiste à réaliser la « montée » de la molécule sur les fibres, doit être réglée pour que la molécule n'atteigne pas les couches plus profondes des fibres kératiniques.

De plus, l'extraction de la molécule « montée » sur les fibres kératiniques doit être effectuée dans les mêmes conditions de pH que l'immersion, pour les raisons précitées. La solution d'extraction contient la quantité de molécules ayant monté en surface sur les fibres kératiniques, quantités correspondant à un délai de surface déterminé. Comme elle est colorée naturellement ou susceptible de l'être après révélation, il est aisé de comparer cette coloration (ou l'absorbance) à celles d'une gamme de solutions de la même molécule à différentes concentrations, chacune de ces colorations étant associée à un état de surface bien déterminé, pour connaître alors l'état de surface des fibres kératiniques que l'on étudie.

La présente invention a donc pour objet un procédé pour évaluer l'état de surface de fibres kératiniques, en particulier de cheveux humains et de poils, caractérisé par le fait que :

a) on immerge un échantillon de fibres kératiniques dans une solution aqueuse d'immersion contenant une molécule qui appartient à la série benzénique ou naphtalénique, qui a un encombrement maximum de $10^{-3}$ µm, qui est suffisamment soluble en milieu aqueux pour permettre la préparation d'une solution contenant au moins 0,01 mole par litre, qui est apte à former, en milieu aqueux, une solution capable d'absorber des radiations dans le visible et/ou l'ultra-violet, naturellement ou après révélation colorimétrique et dont l'absorbance molaire, mesurée à la longueur d'onde du maximum du spectre d'absorption de ladite solution colorée, est au moins égale à 1500, la solution aqueuse d'immersion ayant un pH tel qu'aucune charge ionique n'apparaisse sur la molécule de façon à réaliser une « montée » de la molécule sur lesdites fibres qui soit fonction de l'état de surface de ces dernières ;

b) on retire l'échantillon de fibres kératiniques de ladite solution et on élimine l'excès de ladite solution imbibant les fibres par essorage ou lavage rapide ;

c) on extrait la molécule des fibres dudit échantillon par immersion des fibres dans au moins un bain aqueux dont le pH est sensiblement identique à celui de la solution aqueuse d'immersion pour obtenir une solution d'extraction respectivement colorée ou susceptible d'être colorée par une révélation ;

d) on révèle, s'il y a lieu, la coloration de la solution d'extraction ; et

e) on déduit l'état de surface desdites fibres kératiniques de la quantité de molécules contenue dans ladite solution d'extraction.

A titre d'exemples préférés, on cite :

— comme molécules ne nécessitant pas de révélation : une molécule de formule :

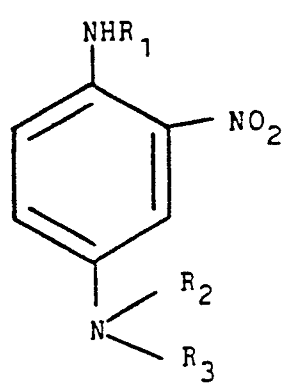

(I)

dans laquelle :

— $R_1$ représente H, $CH_2CH_2OH_2$, $CH_2CH_2NH_2$, $CH_3$ ;

— $R_2$ et $R_3$ représentent indépendamment H et $CH_2CH_2OH$ ;

comme molécules nécessitant une révélation : une molécule prise dans le groupe formé par le dihydroxy-1,3-naphtalène, l'α-naphtol, le diméthyl-2,6 phénol et le sulfate de diamino-2,4 anisole.

Parmi les molécules précitées ne nécessitant pas de révélation, on préfère plus particulièrement la nitro-2 paraphénylène-diamine, désignée dans le Color Index sous la désignation « CI 760700 », le N-(β-hydroxyéthyl) amino-2 N,N-bis-(β-hydroxyéthyl)amino-5 nitrobenzène, mentionné dans le Color Index sous la désignation « HC Blue 2 », le N-(β-aminoéthyl)amino-2 N,N-bis-(β-hydroxyéthyl)amino-5 nitrobenzène, le N-méthylamino-2 N,N-bis-(β-hydroxyéthyl)amino-5 nitrobenzène, et la N-(β-hydroxyéthyl)amino-4 nitro-3 aniline.

Conformément à un mode préféré de mise en œuvre de la présente invention, on utilise, à l'étape a) du procédé, une solution aqueuse de la molécule choisie, qui contient un tiers-solvant, le rapport en poids (partie aqueuse/tiers-solvant) étant au moins égal à 80/20 ; le rapport en poids (partie aqueuse/tiers-solvant) est avantageusement de l'ordre de 90/10. De préférence, on choisit l'éthanol comme tiers-solvant. Le tiers-solvant permet de parfaire la solubilité de la molécule dans la solution utilisée, pour réaliser « la montée » de cette molécule sur les fibres kératiniques. La quantité de tiers-solvant ne peut toutefois dépasser la valeur de 20 % en poids par rapport au poids total de la composition, car une quantité trop importante de tiers-solvant modifie de manière significative l'état de surface de la fibre kératinique par contraction de cette dernière, ce qui perturbe la mise en œuvre correcte du procédé.

Par ailleurs, comme il est nécessaire que, durant toute la mise en œuvre du procédé, la molécule soit maintenue à un pH tel qu'aucune charge ionique, positive ou négative, n'apparaisse, on utilise avantageusement, à l'étape a), une solution aqueuse de la molécule précitée, dont la partie aqueuse est constituée par une solution-tampon. De préférence, on utilise une solution aqueuse, dont le pH se situe, en deçà ou au-delà du pKA de la molécule, à au moins deux unités de pH. Selon les groupements susceptibles d'ionisation portés par la molécule, on utilise avantageusement soit des tampons à pH 10 (carbonate, borate, tampon ammoniacal, etc...), soit des tampons inférieurs à pH 5 (acétate, citrate, etc...).

Conformément à un mode préféré de mise en œuvre de la présente invention, à l'étape a), on réalise l'immersion de l'échantillon de fibres kératiniques à raison d'environ 100 mg de fibres pour 2 à 10 ml de solution pendant au plus 20 minutes, de préférence, pendant un temps compris entre 2 et 5 minutes, et à une température inférieure à 50 °C, de préférence, à une température voisine de l'ambiante.

On a vu que, si l'on immerge une fibre kératinique dans une solution telle que définie ci-dessus, la cinétique de « montée » de la molécule sur la fibre kératinique dépend de l'état de surface de cette dernière. En liaison avec cette observation, la Société déposante a constaté que, si l'immersion de l'étape a) dure trop longtemps (plus de 20 minutes), la quantité de molécule extractible du cheveu est alors sensiblement la même, quel que soit l'état de surface de la fibre, et elle a découvert que, si l'immersion de la fibre kératinique dure moins de 20 minutes, et, de préférence, de 2 à 5 minutes, la quantité de colorant extractible de la fibre kératinique est alors directement en relation avec la qualité de la surface de la cuticule de cette fibre kératinique.

Conformément à un mode préféré de mise en œuvre de la présente invention, on utilise, pour l'extraction de l'étape c), au moins un bain de la même solution-tampon que celle ayant servi à la préparation de la solution d'immersion, ou au moins un bain d'eau amené au même pH que celui de la solution-tampon ayant servi à la préparation de la solution d'immersion. On réalise l'extraction, de préférence, pendant un temps d'au moins 3 minutes, par exemple de 5 minutes ou même davantage.

Conformément à un mode préféré de mise en œuvre de la présente invention, à l'étape e), on compare la solution d'extraction colorée, ou après révélation de sa coloration, à une gamme de dilutions d'une solution concentrée de la molécule dans le même milieu que celui ayant servi à l'extraction, ou bien on analyse la solution d'extraction colorée, ou après révélation de sa coloration, à l'aide d'un spectrophotomètre ou d'un colorimètre calé sur la longueur d'onde au maximum du spectre d'absorption de la solution d'extraction, respectivement colorée ou après révélation de sa coloration.

Ensuite, on exprime les résultats sur une échelle colorimétrique (échelle d'absorbances), sur une échelle de concentrations de la molécule ou sur une échelle arbitraire après étalonnage. Dans ce dernier cas, s'il s'agit en particulier des cheveux, on réalise, par exemple, l'étalonnage sur un cheveu naturel et sur un cheveu fortement sensibilisé. A titre d'exemple, en ce qui concerne les cheveux que l'on trouve sur têtes dans un salon de coiffure, quelle que soit l'expression des résultats, ceux-ci se placent sur une échelle de sensibilisation généralement définie comme suit :

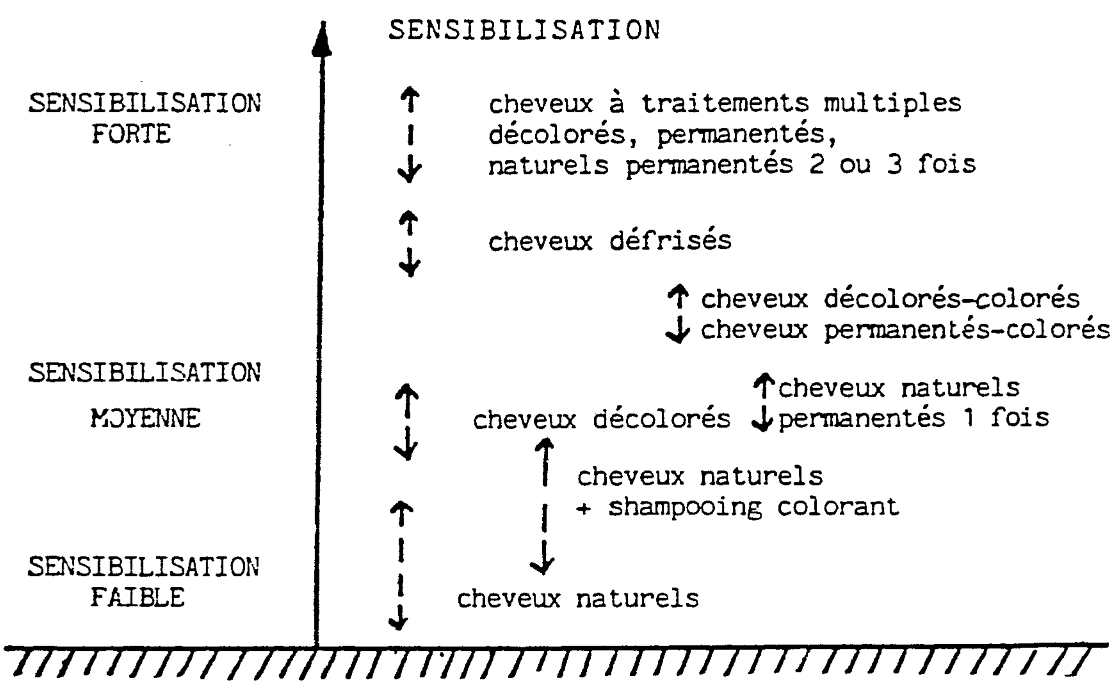

La présente invention, a également pour objet une composition pour la mise en œuvre du procédé ci-dessus défini, cette composition étant caractérisée par le fait qu'elle consiste en une solution-tampon aqueuse contenant une molécule qui appartient à la série benzénique ou naphtalénique, qui a un encombrement maximum de $10^{-3}$ µm, qui est suffisamment soluble en milieu aqueux pour permettre la préparation d'une solution contenant au moins 0,01 mole par litre, qui est apte à former, en milieu aqueux, une solution capable d'absorber des radiations dans le visible et/ou l'ultra-violet, naturellement ou après révélation colorimétrique et dont l'absorbance molaire, mesurée à la longueur d'onde du maximum du spectre d'absorption de ladite solution colorée, est au moins égale à 1500, la solution-tampon aqueuse d'immersion ayant un pH tel qu'aucune charge ionique n'apparaisse sur la molécule.

La molécule présente avantageusement les caractéristiques précédemment indiquées.

Les caractéristiques avantageuses de cette composition et celles de la molécule qu'elle contient ont été également définies ci-dessus.

Pour mieux faire comprendre l'objet de l'invention, on va en décrire ci-après, à titre d'exemples purement illustratifs et non limitatifs, plusieurs modes de mises en œuvre.

## Exemple 1

On dissout 250 mg de nitro-2 paraphénilènediamine dans 10 ml d'éthanol absolu. On complète à 100 ml avec une solution-tampon ammoniacale à pH 10. La concentration en nitro-2 paraphénylènediamine est de 0,016 mole par litre.

On pèse 100 mg de cheveux coupés, d'une longueur d'environ 2 cm. On les introduit dans un bécher de 10 ml. On ajoute 5 ml de la solution de nitro-2 paraphénylènediamine préalablement maintenue à 30 °C. On laisse en contact pendant 2 minutes à 30 °C. On verse le tout sur un entonnoir filtrant cylindro-conique de porosité 4. On tire sous vide pendant 2 minutes en répartissant bien les cheveux sur le fritté avec une baguette de verre.

On transvase les cheveux dans un bécher de 25 ml. On ajoute 10 ml de la solution-tampon ammoniacale à pH 10. On laisse en contact pendant 30 minutes à 30 °C. On transvase la solution dans une fiole jaugée de 25 ml. On répète cette opération une deuxième fois. On complète à 25 ml avec la solution-tampon à pH 10. On lit au spectrophotomètre l'absorbance à 470 nm.

### Gamme d'étalonnage

On prend 2 ml de la solution de nitro-2 paraphénylènediamine. On complète à 25 ml avec la solution-tampon à pH 10. On reprend successivement 1 ml, 2 ml et 3 ml de cette solution et on complète à chaque fois à 25 ml avec la solution-tampon à pH 10. On lit l'absorbance de chaque solution à 470 nm. On trace la courbe d'étalonnage.

### Résultats

On exprime les résultats sur une échelle colorimétrique ou sur une échelle de concentrations de la molécule non chargée, comme indiqué dans le tableau suivant, d'où l'on déduit que le cheveu étudié appartient à l'un des types 1 à 9, ces types étant définis après le tableau.

Tableau

Absorbance molaire de la nitro-2 paraphénylènediamine : 4000

| | cheveu 1 | cheveu 2 | cheveu 3 | cheveu 4 | cheveu 5 | cheveu 6 | cheveu 7 | cheveu 8 | cheveu 9 |
|---|---|---|---|---|---|---|---|---|---|
| Absorbance de la solution | 0,29 | 0,51 | 0,57 | 0,67 | 0,52 | 0,92 | 0,89 | 1,01 | 1,24 |
| Concentration (µmoles de colorant pour 100 mg cheveu) | 1,8 | 3,2 | 3,6 | 4,2 | 3,3 | 6,0 | 5,8 | 6,3 | 7,8 |

Cheveu 1 : cheveu naturel
Cheveu 2 : cheveu décoloré faible
Cheveu 3 : cheveu décoloré moyen
Cheveu 4 : cheveu décoloré fort
Cheveu 5 : cheveu naturel + 1 permanente
Cheveu 6 : cheveu naturel + 3 permanentes
Cheveu 7 : cheveu décoloré faible + 1 permanente
Cheveu 8 : cheveu décoloré moyen + 1 permanente
Cheveu 9 : cheveu décoloré fort + 1 permanente

Exemple 2

On dissout 250 mg de nitro-2 paraphénylènediamine dans 10 ml d'éthanol absolu. On complète à 100 ml avec une solution-tampon ammoniacale à pH 10.

Une seringue de 5 ml en matière plastique, d'un diamètre de 1 cm est modifiée par l'introduction dans le fond du corps de la seringue d'une grille en matière plastique. Le bout de la seringue, recevant en utilisation normale l'aiguille, est supprimé afin d'éliminer tout volume mort. On introduit, dans la seringue, une quantité de cheveux comprise entre 20 et 30 mg. Ces cheveux sont prélevés sur tête, sans pesée, par comparaison avec une mèche de référence. On amène le piston au zéro de la seringue. On aspire 2 ml de solution colorée de nitro-2 paraphénylènediamine en tirant le piston jusqu'au repère de la seringue. On laisse en contact exactement pendant 2 minutes à la température ambiante. On rejette le liquide. On essuie le bout de la seringue. On aspire 5 ml de solution-tampon ammoniacale à pH 10. On rejette immédiatement cette solution. On essuie à nouveau le bout de la seringue. On aspire 5 ml de solution-tampon ammoniacale à pH 10 et on laisse en contact pendant 5 minutes à température ambiante. On recueille le liquide dans un tube à essai.

On compare la coloration à deux tubes de référence A et B d'un colorant rouge. Le tube de référence A contient 5 ml d'une solution de 7,7 mg de nitro-2 paraphénylènediamine par litre de tampon ammoniacal à pH 10 (tube scellé) ; l'absorbance est de 0,20 ; ce tube de référence A représente la limite supérieure de l'état 1. Le tube de référence B contient 5 ml d'une solution de 26,5 mg de nitro-2 paraphénylènediamine par litre de tampon ammoniacal à pH 10 (tube scellé) ; l'absorbance est de 0,69 ; ce tube de référence B représente la limite inférieure de l'état 3.

Toute coloration comprise entre les tubes de référence A et B est considérée comme appartenant à l'état 2.

Les résultats sont exprimés de la façon suivante :

— l'état 1 correspond à un cheveu naturel, c'est-à-dire à un cheveu peu sensibilisé ;

— l'état 2 correspond à un cheveu ayant subi un traitement cosmétique simple (une décoloration ou une permanente ou une coloration), c'est-à-dire à un cheveu moyennement sensibilisé ;

— l'état 3 correspond à un cheveu ayant subi des traitements cosmétiques multiples répétés ou combinés (décoloration + permanente ou permanentes répétées), c'est-à-dire à un cheveu fortement sensibilisé.

Cette classification est fort utile, par exemple pour le coiffeur qui peut ainsi facilement régler les conditions opératoires d'une décoloration (temps de pose-concentration de l'oxydant...) ou d'une permanente (force du liquide réducteur) en fonction de la qualité du cheveu de la cliente.

Exemple 3

On dissout 830 mg de colorant « HC Blue 2 » dans 10 ml d'éthanol absolu. On complète à 100 ml avec une solution-tampon ammoniacale à pH 10. La concentration en « HC Blue 2 » est de 0,029 mole par litre.

On pèse 100 mg de cheveux coupés d'une longueur d'environ 2 cm. On les introduit dans un bécher de 10 ml. On ajoute 5 ml de la solution de « HC Blue 2 », préalablement maintenue à 30 °C. On laisse en contact pendant 2 minutes à 30 °C. On verse le tout sur un entonnoir filtrant cylindro-conique de porosité 4. On tire sous vide pendant 2 minutes en répartissant bien les cheveux sur le fritté avec une baguette de verre.

On transvase les cheveux dans un bécher de 25 ml. On ajoute 10 ml de la solution-tampon ammoniacale à pH 10. On laisse en contact pendant 30 minutes à 30 °C. On transvase la solution dans une fiole jaugée de 25 ml. On répète cette opération une deuxième fois. On complète à 25 ml avec la solution-tampon à pH 10. On lit au spectrophotomètre l'absorbance à 540 nm.

On peut exprimer les résultats sur une échelle de sensibilisation définie de la manière suivante :

| | |
|---|---|
| Cheveu naturel, très peu sensibilisé : | note 10 - absorbance : 0,15 |
| Cheveu très sensibilisé : | note 100 - absorbance : 1,05 |

Le degré de sensibilisation du cheveu est alors obtenu par la formule suivante :

$$S = 100 \left( \frac{100 \times A}{P\,E} \right) - K$$

— dans laquelle A représente l'absorbance de la solution d'extraction,

— PE la prise d'essai du cheveu en mg soit environ 100,

— K une constante qui est égale à 5 dans les conditions de ce test.

En conclusion, on exprime les résultats sur une échelle colorimétrique ou sur cette échelle de

sensibilisation, comme indiqué dans le tableau suivant, d'où l'on déduit que le cheveu étudié appartient à l'un des types 1 à 9 qui ont été définis à l'exemple 1.

Tableau

Absorbance molaire du « HC Blue 2 » : 3500

| | Cheveu 1 | Cheveu 2 | Cheveu 3 | Cheveu 4 | Cheveu 5 | Cheveu 6 | Cheveu 7 | Cheveu 8 | Cheveu 9 |
|---|---|---|---|---|---|---|---|---|---|
| Absorbance de la solution | 0,15 | 0,22 | 0,30 | 0,42 | 0,23 | 0,53 | 0,50 | 0,77 | 1,10 |
| Degré de sensibilisation | 10 | 17 | 25 | 37 | 18 | 48 | 45 | 72 | 105 |

Exemple 4

On dissout 830 mg de colorant « HC Blue 2 » dans 10 ml d'éthanol absolu. On complète à 100 ml avec la solution-tampon ammoniacale à pH 10.

On introduit environ 25 mg de cheveux (entre 20 et 30 mg de cheveux pris sur tête), sans pesée, par comparaison à une mèche de référence, dans une seringue modifiée comme pour l'exemple 2. On aspire 2 ml de solution colorée de « HC Blue 2 » en tirant le piston jusqu'au repère de la seringue. On laisse en contact exactement pendant 2 minutes à la température ambiante. On rejette le liquide et on essuie le bout de la seringue. On aspire 5 ml de solution-tampon ammoniacale à pH 10. On rejette immédiatement cette solution et on essuie le bout de la seringue. On aspire 5 ml de solution-tampon ammoniacale à pH 10 et on laisse en contact 5 minutes à la température ambiante. On recueille le liquide dans un tube à essai.

On compare la coloration à une règle colorée préparée une fois pour toutes, comportant un dégradé de bleu (nuance du colorant « HC Blue 2 »).

La limite supérieure de l'état 1 est placée au niveau de la règle à une intensité de coloration comparable à une solution de 16,4 mg de « HC Blue 2 » par litre de tampon ammoniacal à pH 10 (absorbance : 0,20).

La limite inférieure de l'état 3 est placée au niveau de la règle à une intensité de coloration comparable à une solution de 43,9 mg de « HC Blue 2 » par litre de tampon ammoniacal à pH 10 (absorbance : 0,54).

Les résultats sont similaires à ceux obtenus à l'exemple 2.

Exemple 5

Le mode opératoire est le même que celui décrit pour l'exemple 2. La seule modification intervient sur la durée de l'extraction en fin de test. Cette durée est ici de 3 minutes à la température ambiante. Afin de conserver la même répartition des cheveux dans les états 1, 2 et 3, il est nécessaire de modifier comme suit les tubes de référence A et B :

— le tube de référence A a maintenant une absorbance de 0,16 (5 ml d'une solution de 6,1 mg de nitro-2 paraphénylènediamine par litre de tampon ammoniacal) ;

— le tube de référence B a maintenant une absorbance de 0,52 (5 ml d'une solution de 19,9 mg de nitro-2 paraphénylènediamine par litre de tampon ammoniacal).

Exemple 6

Le mode opératoire est le même que celui décrit à l'exemple 2. La seule modification intervient sur la durée de contact cheveu/solution colorée (première partie du test). Cette durée est ici de 1 minute (à température ambiante). La durée de l'extraction en fin de test est semblable à celle de l'exemple 2, soit 5 minutes. Afin de conserver la même répartition des cheveux dans les états 1, 2 et 3, il est nécessaire de modifier comme suit les tubes de références A et B :

— Le tube de référence A a maintenant une absorbance de 0,14 (5 ml d'une solution de 5,4 mg de nitro-2 paraphénylènediamine par litre de tampon ammoniacal).

— Le tube de référence B a maintenant une absorbance de 0,40 (5 ml d'une solution de 15,3 mg de nitro-2 paraphénylènediamine par litre de tampon ammoniacal).

Exemple 7

On dissout 800 mg de N-(β-aminoéthyl)amino-2 N,N-bis-(β-hydroxyéthyl)amino-5 nitrobenzène dans 100 ml de solution-tampon ammoniacale à pH 10. La concentration en N-(β-aminoéthyl)amino-2 N,N-bis-(β-hydroxyéthyl)amino-5 nitrobenzène est de 0,028 mole par litre.

On introduit une quantité de cheveux comprise entre 30 et 60 mg dans la seringue modifiée comme pour l'exemple 2. On aspire 2 ml de solution colorée préparée précédemment en tirant le piston jusqu'au repère de la seringue. On laisse en contact exactement pendant 2 minutes à la température ambiante. On rejette le liquide et on essuie le bout de la seringue. On aspire 5 ml de solution-tampon ammoniacale à pH 10. On rejette immédiatement cette solution et on essuie le bout de la seringue. Ensuite on aspire 5 ml de solution-tampon ammoniacale à pH 10 et on laisse en contact pendant 5 minutes à la température ambiante. On recueille le liquide dans un tube à essai.

On compare la coloration à deux tubes de références A et B (coloration bleue). Absorbance molaire du N-(β-aminoéthyl)amino-2 N,N-bis-(β-hydroxyéthyl)amino-5 nitro-benzène : 2500. Le tube A contient 5 ml d'une solution de 18 mg de colorant par litre de tampon ammoniacal à pH 10 (tube scellé) ; l'absorbance est de 0,16 ; ce tube de référence A représente la limite supérieure de l'état 1. Le tube de référence B contient 5 ml d'une solution de 42,6 mg de colorant par litre de tampon ammoniacal à pH 10 (tube scellé) ; l'absorbance est de 0,38 ; ce tube de référence B représente la limite inférieure de l'état 3.

Toute coloration comprise entre les tubes de références A et B est considérée comme appartenant à l'état 2.

Les résultats sont similaires à ceux obtenus à l'exemple 2.

Exemple 8

On dissout 300 mg de N-(β-hydroxyéthyl)amino-4 nitro-3 aniline dans 10 ml d'éthanol absolu. On complète à 100 ml avec une solution-tampon ammoniacale à pH 10. La concentration en N-(β-hydroxyéthyl)amino-4 nitro-3 aniline est de 0,015 mole par litre.

On pèse 100 mg de cheveux coupés d'une longueur d'environ 2 cm. On les introduit dans un bécher de 10 ml. On ajoute 5 ml de la solution de colorant préalablement maintenue à 30 °C. On laisse en contact pendant 2 minutes à 30 °C. On verse le tout sur un entonnoir filtrant cylindro-conique de porosité 4. On tire sous vide pendant 2 minutes en répartissant bien les cheveux sur le fritté avec une baguette de verre.

On transvase les cheveux dans un bécher de 25 ml. On ajoute 10 ml de la solution-tampon ammoniacale à pH 10. On laisse en contact pendant 30 minutes à 30 °C. On transvase la solution dans une fiole jaugée de 25 ml. On répète cette opération une deuxième fois. On complète à 25 ml avec la solution-tampon à pH 10. On lit au spectrophotomètre l'absorbance à 505 nm.

Gamme d'étalonnage

On prend 2 ml de la solution de N-(β-hydroxyéthyl)amino-4 nitro-3 aniline. On complète à 20 ml avec la solution-tampon à pH 10. On reprend successivement 0,5 ml, 1 ml et 2 ml de cette solution et on complète à chaque fois à 25 ml de la solution-tampon à pH 10. On lit l'absorbance de chaque solution à 505 nm. On trace la courbe d'étalonnage.

Résultats

On exprime les résultats sur une échelle colorimétrique ou sur une échelle de concentrations, comme indiqué dans le tableau suivant, d'où l'on déduit que le cheveu étudié appartient à l'un des types 1 à 9 qui ont été définis à l'exemple 1.

Tableau

Absorbance molaire du N-(β-hydroxyéthyl)amino-4 nitro-3 aniline : 4200

| | Cheveu 1 | Cheveu 2 | Cheveu 3 | Cheveu 4 | Cheveu 5 | Cheveu 6 | Cheveu 7 | Cheveu 8 | Cheveu 9 |
|---|---|---|---|---|---|---|---|---|---|
| Absorbance de la solution | 0,16 | 0,33 | 0,37 | 0,41 | 0,35 | 0,50 | 0,48 | 0,55 | 0,64 |
| Concentration (µmoles de colorant pour 100 mg de cheveux) | 1 | 2 | 2,2 | 2,4 | 2,1 | 3,1 | 3 | 3,4 | 4 |

Exemple 9

On dissout 800 mg de N-méthylamino-2 N,N-bis-(β-hydroxyéthyl)amino-5 nitrobenzène dans 10 ml d'éthanol absolu. On complète à 100 ml de solution-tampon ammoniacale à pH 10. La concentration en N-méthylamino-2 N,N-bis-(β-hydroxyéthyl)amino-5 nitrobenzène est de 0,031 mole par litre.

On introduit 40 mg de cheveux dans la seringue modifiée comme pour l'exemple 2. On aspire 5 ml de la solution colorée préparée précédemment en tirant le piston jusqu'au repère de la seringue. On laisse en contact pendant exactement 2 minutes à la température ambiante. On rejette le liquide et on essuie le bout de la seringue. On aspire 2 ml de tampon ammoniacal à pH 10. On rejette immédiatement cette solution et on essuie le bout de la seringue. On répète cette opération une seconde fois, à savoir l'aspiration de 2 ml de solution-tampon et rejet immédiat. Ensuite, on aspire 5 ml de solution-tampon ammoniacale à pH 10 et on laisse en contact pendant 15 minutes à la température ambiante. On recueille le liquide dans un tube à essai. On aspire à nouveau 5 ml de solution-tampon ammoniacale à pH 10 et on laisse en contact pendant 5 minutes à la température ambiante. On recueille le liquide dans le même tube à essai que précédemment.

On compare la coloration à deux tubes de référence A et B (coloration bleue). Absorbance molaire du N-méthylamino-2, N,N-bis-(β-hydroxyéthyl)amino-5 nitrobenzène : 3800.

Le tube de référence A, qui représente la limite supérieure de l'état 1, contient 10 ml d'une solution de 9,4 mg de colorant par litre de tampon ammoniacal à pH 10 (tube scellé) ; l'absorbance est de 0,14.

Le tube de référence B, qui représente la limite inférieure de l'état 3, contient 5 ml d'une solution de 18,1 mg de colorant par litre de tampon ammoniacal à pH 10 (tube scellé) ; l'absorbance est de 0,27.

Toute coloration comprise entre les tubes de références A et B est considérée comme appartenant à l'état 2.

Les résultats sont similaires à ceux obtenus à l'exemple 2.

Exemple 10

On dissout 244 mg de diméthyl-2,6 phénol dans 5 ml d'éthanol absolu. On complète à 100 ml avec une solution-tampon acétate à pH 4,7. La concentration en diméthyl-2,6 phénol est de 0,02 mole par litre.

On pèse 100 mg de cheveux coupés d'une longueur d'environ 2 cm. On les introduit dans un bécher de 10 ml. On ajoute 5 ml de la solution de diméthyl-2,6 phénol préalablement maintenue à 30 °C. On laisse en contact pendant 2 minutes à 30 °C. On verse le tout sur un entonnoir filtrant cylindro-conique de porosité 4. On tire sous vide pendant 2 minutes en répartissant bien les cheveux sur le fritté avec une baguette de verre. On transvase les cheveux dans un bécher de 25 ml. On ajoute 10 ml d'eau. On laisse en contact pendant 30 minutes à 30 °C. On transvase la solution dans une fiole jaugée de 25 ml. On répète cette opération une deuxième fois et on complète à 25 ml avec de l'eau.

Révélation

A 1 ml de la solution d'extraction, on ajoute successivement 2 ml de tampon ammoniacal à pH 10, 2 ml d'une solution contenant 34,6 mg de paraphénylènediamine par litre d'eau et 0,2 ml d'une solution contenant 3 g de ferricyanure de potassium par litre d'eau. On laisse la coloration se développer pendant 10 minutes. On lit au spectrophotomètre l'absorbance à 525 nm par rapport à un témoin préparé comme précédemment, mais en remplaçant la solution d'extraction par 1 ml d'eau.

Gamme d'étalonnage

On dissout 48,8 mg de diméthyl-2,6 phénol dans 50 ml d'eau. On prend trois fois de suite 1 ml de cette solution et on complète successivement à 25 ml, 50 ml et 100 ml avec de l'eau.

On prend successivement 1 ml de ces trois dernières solutions, on ajoute dans chaque cas 2 ml de tampon ammoniacal à pH 10, puis 2 ml de la solution de paraphénylènediamine et 0,2 ml de la solution de ferricyanure de potassium.

On laisse la coloration se développer pendant 10 minutes. On lit au spectrophotomètre l'absorbance à 525 nm par rapport à un témoin préparé en remplaçant par 1 ml d'eau les solutions de diméthyl-2,6 phénol. On trace la courbe d'étalonnage.

Résultats

On exprime les résultats sur une échelle colorimétrique ou sur une échelle de concentrations, comme indiqué dans le tableau suivant, d'où l'on déduit que le cheveu étudié appartient à l'un des types 1 à 9 qui ont été définis à l'exemple 1.

(Voir tableau page 10)

Tableau

Absorbance molaire du diméthyl-2,6 phénol dans les conditions de révélation : 1 750.

| | Cheveu 1 | Cheveu 2 | Cheveu 3 | Cheveu 4 | Cheveu 5 | Cheveu 6 | Cheveu 7 | Cheveu 8 | Cheveu 9 |
|---|---|---|---|---|---|---|---|---|---|
| Absorbance de la solution | 0,20 | 0,25 | 0,32 | 0,36 | 0,33 | 0,51 | 0,46 | 0,57 | 0,67 |
| Concentration (µmoles de réactif pour 100 mg de cheveux) | 2,9 | 3,6 | 4,6 | 5,2 | 4,7 | 7,3 | 6,7 | 8,2 | 9,6 |

Exemple 11

On dissout 288 mg d'α-naphtol dans 15 ml d'éthanol absolu. On complète à 100 ml avec une solution-tampon acétate à pH 4,7. La concentration en α-naphtol est de 0,02 mole par litre.

On pèse 100 mg de cheveux coupés d'une longueur d'environ 2 cm. On les introduit dans un bécher de 10 ml. On ajoute 5 ml de la solution d'α-naphtol préalablement maintenue à 40 °C. On laisse en contact pendant 2 minutes à 40 °C. On verse le tout sur un entonnoir filtrant cylindro-conique de porosité 4. On tire sous vide pendant 2 minutes en répartissant bien les cheveux sur le fritté avec une baguette de verre. On transvase les cheveux dans un bécher de 25 ml. On ajoute 10 ml d'eau et on laisse en contact pendant 30 minutes à 40 °C. On transvase la solution dans une fiole jaugée de 25 ml. On répète cette opération une deuxième fois et on complète à 25 ml avec de l'eau.

Révélation

Dans un tube à réaction, on place successivement 2 ml d'acide sulfurique concentré, 0,1 ml d'une solution de glyoxal à 5 g pour 100 ml d'eau préparée au moment de l'emploi, et 1 ml de la solution à révéler. On lit au spectrophotomètre l'absorbance à 615 nm, par rapport à un témoin préparé comme précédemment, mais en remplaçant la solution à révéler par 1 ml d'eau.

Gamme d'étalonnage

On dissout 57,5 ml d'α-naphtol dans 2 ml d'éthanol absolu et de l'eau jusqu'à un volume total de 50 ml. On prend trois fois de suite 1 ml de cette solution et on complète successivement à 25 ml, 50 ml et 100 ml avec de l'eau. Dans un tube à réaction, on place successivement 2 ml d'acide sulfurique concentré, 0,1 ml de la solution de glyoxal et 1 ml d'une des trois solutions précédentes. Dans deux autres tubes à réaction, on opère de même avec les deux autres solutions précédentes. On lit au spectrophotomètre l'absorbance à 615 nm, par rapport à un témoin préparé en remplaçant par 1 ml d'eau, les solutions d'α-naphtol. On trace la courbe d'étalonnage.

Résultats

On exprime les résultats sur une échelle colorimétrique ou sur une échelle de concentrations, comme indiqué dans le tableau suivant, d'où l'on déduit que le cheveu étudié appartient à l'un des types 1 à 9 qui ont été définis à l'exemple 1.

(Voir tableau page 11)

Tableau

Absorbance molaire de l'α-naphtol dans les conditions de révélation : 4 500

| | Cheveu 1 | Cheveu 2 | Cheveu 3 | Cheveu 4 | Cheveu 5 | Cheveu 6 | Cheveu 7 | Cheveu 8 | Cheveu 9 |
|---|---|---|---|---|---|---|---|---|---|
| Absorbance de la solution | 0,53 | 0,75 | 0,81 | 0,92 | 0,76 | 1,23 | 1,16 | 1,30 | 1,54 |
| Concentration (µmoles d'α-naphtol pour 100 mg de cheveux) | 2,8 | 4,0 | 4,3 | 4,9 | 4,1 | 6,6 | 6,2 | 6,9 | 8,2 |

Exemple 12

On dissout 526 mg de sulfate de diamini-2,4 anisole (molécule cristallisée avec 1,5 moles d'$H_2O$) dans 100 ml de tampon ammoniacal à pH 10. La concentration en sulfate de diamino-2,4 anisole est de 0,02 mole par litre.

On pèse 100 mg de cheveux coupés d'une longueur d'environ 2 cm. On les introduit dans un bécher de 10 ml. On ajoute 5 ml de la solution de sulfate de diamino-2,4 anisole, préalablement maintenue à 30 °C. On laisse en contact pendant 2 minutes à 30 °C. On verse le tout sur un entonnoir filtrant cylindro-conique de porosité 4. On tire sous vide pendant 2 minutes en répartissant bien les cheveux sur le fritté avec une baguette de verre. On transverse les cheveux dans un bécher de 25 ml. On ajoute 10 ml de tampon ammoniacal à pH 10. On laisse en contact pendant 30 minutes à 30 °C. On transverse la solution dans une fiole jaugée de 25 ml. On répète cette opération une deuxième fois et on complète à 25 ml avec du tampon ammoniacal à pH 10.

Révélation

A 2 ml de la solution d'extraction, on ajoute successivement 2 ml de tampon ammoniacal à pH 10,2 ml d'une solution contenant 34,6 mg de paraphénylènediamine par litre d'eau, et 0,15 ml d'une solution contenant 3 g de ferricyanure de potassium par litre d'eau. On laisse la coloration se développer pendant 10 minutes. On lit au spectrophotomètre l'absorbance à 515 nm, par rapport à un témoin préparé comme précédemment, mais en remplaçant la solution d'extraction par 2 ml de tampon ammoniacal à pH 10.

Gamme d'étalonnage

On dissout 210,4 mg de sulfate de diamino-2,4 anisole dans 100 ml de tampon ammoniacal à pH 10. On prend trois fois de suite, 1 ml de cette solution, et on complète successivement à 25 ml, 50 ml et 100 ml avec du tampon ammoniacal à pH 10. Dans un tube à réaction, on place successivement 2 ml d'une des trois solutions précédentes, 2 ml de tampon ammoniacal à pH 10, 2 ml de la solution de paraphénylènediamine et 0,15 ml de la solution de ferricyanure de potassium. Dans deux autres tubes à réaction, on opère de même avec les deux autres solutions précédentes.

On laisse la coloration se développer pendant 10 minutes. On lit au spectrophotomètre l'absorbance à 515 nm, par rapport à un témoin préparé comme précédemment, mais en remplaçant par 2 ml de tampon ammoniacal à pH 10 les solutions de sulfate de diamino-2,4 anisole. On trace la courbe d'étalonnage.

Résultats

On exprime les résultats sur une échelle colorimétrique ou sur une échelle de concentrations, comme indiqué dans le tableau suivant, d'où l'on déduit que le cheveu étudié appartient à l'un des types 1 à 9 qui ont été définis à l'exemple 1.

(Voir tableau page 12)

Tableau

Absorbance molaire du sulfate de diamino-2,4 anisole dans les conditions de révélation : 2 300

| | Cheveu 1 | Cheveu 2 | Cheveu 3 | Cheveu 4 | Cheveu 5 | Cheveu 6 | Cheveu 7 | Cheveu 8 | Cheveu 9 |
|---|---|---|---|---|---|---|---|---|---|
| Absorbance de la solution | 0,18 | 0,21 | 0,25 | 0,27 | 0,26 | 0,42 | 0,39 | 0,46 | 0,51 |
| Concentration (µmoles de réactif pour 100 mg de cheveux) | 1,9 | 2,2 | 2,6 | 2,8 | 2,7 | 4,2 | 4,1 | 4,8 | 5,3 |

Exemple 13

On dissout 256 mg de dihydroxy-1,3 naphtalène dans 10 ml d'éthanol absolu. On complète à 100 ml avec une solution-tampon acétate à pH 4,7. La concentration en dihydroxy-1,3 naphtalène est de 0,016 mole par litre. Cette solution est préparée au moment de l'emploi ; elle est maintenue à 30 °C à l'abri de la lumière.

On pèse 100 mg de cheveux coupés d'une longueur d'environ 2 cm. On les introduit dans un bécher de 10 ml. On ajoute 5 ml de la solution de dihydroxy-1,3 naphtalène. On laisse en contact pendant 2 minutes à 30 °C. On verse le tout sur un entonnoir filtrant cylindro-conique de porosité 4. On tire sous vide pendant 2 minutes en répartissant bien les cheveux sur le fritté avec une baguette de verre. On transvase les cheveux dans un bécher de 25 ml. On ajoute 10 ml d'eau. On laisse en contact pendant 30 minutes à 30 °C. On transvase la solution dans une fiole jaugée de 25 ml. On répète cette opération une deuxième fois et on complète à 25 ml avec de l'eau.

Révélation

On mélange, avant emploi, un volume de solution de $FeCl_3$, 6 $H_2O$ à 3 g par litre d'HCl 0,4N, 1 volume de solution de ferricyanure de potassium à 3 g par litre d'eau et 2 volumes d'eau. On maintient ce réactif à l'abri de la lumière. Dans un tube à réaction, on place 0,5 ml de la solution d'extraction et 4 ml du réactif. On laisse la coloration se stabiliser pendant 10 minutes à l'obscurité. On lit au spectrophotomètre l'absorbance à 715 nm, par rapport à un témoin préparé comme précédemment mais en remplaçant la solution d'extraction par 0,5 ml d'eau.

Gamme d'étalonnage

On dissout 64 mg de dihydroxy-1,3 naphtalène dans 50 ml d'eau. On prend trois fois de suite 1 ml de cette solution et on complète successivement à 25 ml, 50 ml et 100 ml avec de l'eau. On prend successivement 0,5 ml de ces trois dernières solutions. On ajoute, dans chaque cas, 4 ml de réactif ($FeCl_3$/ferricyanure de potassium). On laisse la coloration se stabiliser pendant 10 minutes à l'obscurité. On lit au spectrophotomètre l'absorbance à 715 nm, par rapport à un témoin préparé en remplaçant par 0,5 ml d'eau, les solutions de dihydroxy-1,3 naphtalène. On trace la courbe d'étalonnage.

Résultats

On exprime les résultats sur une échelle colorimétrique ou sur une échelle de concentrations, comme indiqué dans le tableau suivant, d'où l'on déduit que le cheveu étudié appartient à l'un des types 1 à 9 qui ont été définis à l'exemple 1.

(Voir tableau page 13)

Tableau

Absorbance molaire du dihydroxy-1,3 naphtalène dans les conditions de révélation : 2 500

| | Cheveu 1 | Cheveu 2 | Cheveu 3 | Cheveu 4 | Cheveu 5 | Cheveu 6 | Cheveu 7 | Cheveu 8 | Cheveu 9 |
|---|---|---|---|---|---|---|---|---|---|
| Absorbance de la solution | 0,38 | 0,50 | 0,68 | 0,77 | 0,70 | 1,1 | 1,0 | 1,23 | 1,6 |
| Concentration (μmoles de réactif pour 100 mg de cheveux ) | 2,4 | 3,1 | 4,3 | 4,9 | 4,4 | 6,9 | 6,3 | 7,7 | 10,0 |

## Revendications

1. Procédé pour évaluer l'état de surface de fibres kératiniques, en particulier, de cheveux humains et de poils, caractérisé par le fait que

a) on immerge un échantillon de fibres kératiniques dans une solution aqueuse d'immersion contenant une molécule qui appartient à la série benzénique ou naphtalénique, qui a un encombrement maximum de $10^{-3}$ μm, qui est suffisamment soluble en milieu aqueux pour permettre la préparation d'une solution contenant au moins 0,01 mole par litre, qui est apte à former, en milieu aqueux, une solution capable d'absorber des radiations dans le visible et/ou l'ultra-violet, naturellement ou après révélation colorimétrique et dont l'absorbance molaire, mesurée à la longueur d'onde du maximum du spectre d'absorption de ladite solution colorée, est au moins égale à 1 500, la solution aqueuse d'immersion ayant un pH tel qu'aucune charge ionique n'apparaisse sur la molécule de façon à réaliser une « montée » de la molécule sur lesdites fibres qui soit fonction de l'état de surface de ces dernières ;

b) on retire l'échantillon de fibres kératiniques de ladite solution et on élimine l'excès de ladite solution imbibant les fibres par essorage ou lavage rapide ;

c) on extrait la molécule des fibres dudit échantillon par immersion des fibres dans au moins un bain aqueux dont le pH est sensiblement identique à celui de la solution aqueuse d'immersion pour obtenir une solution d'extraction respectivement colorée ou susceptible d'être colorée par une révélation ;

d) on révèle, s'il y a lieu, la coloration de la solution d'extraction ; et

e) on déduit l'état de surface desdites fibres kératiniques de la quantité de molécules contenue dans ladite solution d'extraction.

2. Procédé selon la revendication 1, caractérisé par le fait qu'on choisit, comme molécule ne nécessitant pas de révélation, une molécule de formule :

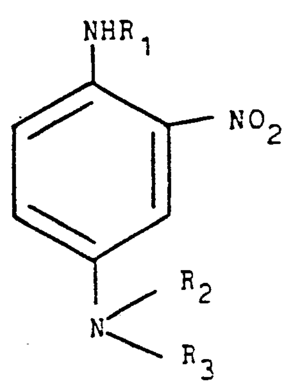

(I)

formule dans laquelle :

— $R_1$ représente H, $CH_2CH_2OH$, $CH_2CH_2NH_2$, $CH_3$ ; et

— $R_2$ et $R_3$ représentent indépendamment H et $CH_2CH_2OH$.

3. Procédé selon la revendication 1, caractérisé par le fait qu'on choisit, comme molécule nécessitant une révélation, une molécule prise dans le groupe formé par le dihydroxy-1,3-naphtalène, l'α-naphtol, le diméthyl-2,6 phénol et le sulfate de diamino-2,4 anisole.

4. Procédé selon l'une des revendications 1 à 3, caractérisé par le fait qu'à l'étape a) on utilise une

solution aqueuse de la molécule choisie qui contient un tiers-solvant comme l'éthanol, le rapport en poids (partie aqueuse/tiers solvant) étant au moins égal à 80/20, et notamment de l'ordre de 90/10.

5. Procédé selon l'une des revendications 1 à 4, caractérisé par le fait qu'à l'étape a), on utilise une solution aqueuse de la molécule choisie, solution dont la partie aqueuse est constituée par une solution-tampon.

6. Procédé selon la revendication 5, caractérisé par le fait qu'on utilise une solution aqueuse dont le pH se situe, en deçà ou au-delà du pKA de la molécule, à au moins 2 unités de pH.

7. Procédé selon l'une des revendications 1 à 6, caractérisé par le fait qu'à l'étape a), on réalise l'immersion de l'échantillon des fibres kératiniques à raison d'environ 100 mg de fibres pour 2 à 10 ml de solution, pendant au plus 20 minutes, de préférence pendant un temps compris entre 2 et 5 minutes, et à une température inférieure à 50 °C, de préférence à une température voisine de l'ambiance.

8. Procédé selon les revendications 1 et 5 prises simultanément, caractérisé par le fait qu'on utilise pour l'extraction de l'étape c) au moins un bain de la même solution-tampon que celle ayant servi à la préparation de la solution d'immersion, ou au moins un bain d'eau amené au même pH que celui de la solution-tampon ayant servi à la préparation de la solution d'immersion.

9. Procédé selon l'une des revendications 1 à 8, caractérisé par le fait qu'on réalise l'extraction de l'étape c) pendant un temps d'au moins 3 minutes.

10. Procédé selon l'une des revendications 1 à 9, caractérisé par le fait qu'à l'étape e), on compare la solution d'extraction colorée ou après révélation de sa coloration, à une gamme de dilution d'une solution concentrée de la molécule dans le même milieu que celui ayant servi à l'extraction, ou bien on analyse la solution d'extraction colorée ou après révélation de sa coloration à l'aide d'un spectrophotomètre ou d'un colorimètre calé sur la longueur d'onde du maximum du spectre d'absorption de la solution d'extraction respectivement colorée ou après révélation de sa coloration.

11. Procédé selon l'une des revendications 1 à 10, caractérisé par le fait qu'à l'étape g), on exprime les résultats sur une échelle colorimétrique, sur une échelle de concentration de la molécule ou sur une échelle arbitraire après étalonnage.

12. Composition pour la mise en œuvre du procédé selon l'une des revendications 1 à 11, caractérisé par le fait qu'elle consiste en une solution-tampon aqueuse contenant une molécule qui appartient à la série benzénique ou naphtalénique, qui a un encombrement maximum de $10^{-3}$ µm, qui est suffisamment soluble en milieu aqueux pour permettre la préparation d'une solution contenant au moins 0,01 mole par litre, qui est apte à former, en milieu aqueux, une solution capable d'absorber des radiations dans le visible et/ou l'ultra-violet, naturellement ou après révélation colorimétrique et dont l'absorbance molaire, mesurée à la longueur d'onde du maximum du spectre d'absorption de ladite solution colorée, est au moins égale à 1 500, la solution-tampon aqueuse d'immersion ayant un pH tel qu'aucune charge ionique n'apparaisse sur la molécule.

13. Composition selon la revendication 12, caractérisée par le fait que les molécules ne nécessitant pas de révélation sont choisies parmi celles représentées par la formule :

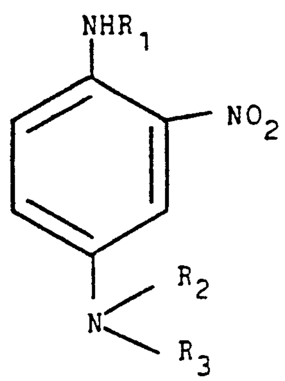

(I)

formule dans laquelle :

— $R_1$ représente H, $CH_2CH_2OH$, $CH_2CH_2NH_2$, $CH_3$ ; et

— $R_2$ et $R_3$ représentent indépendamment H et $CH_2CH_2OH$.

14. Composition selon la revendication 12, caractérisée par le fait que les molécules nécessitant une révélation sont prises dans le groupe formé par le dihydroxy-1,3 naphtalène, l'α-naphtol, le diméthyl-2,6 phénol et le sulfate de diamino-2,4 anisole.

15. Composition selon l'une des revendications 12 à 14, caractérisée par le fait qu'elle contient un tiers-solvant comme l'éthanol, le rapport en poids partie aqueuse/tiers solvant étant au moins égal à 80/20, et notamment de l'ordre de 90/10.

16. Composition selon l'une des revendications 12 à 15, caractérisé par le fait que la partie aqueuse de la solution est constituée par une solution-tampon ayant un pH qui se situe, en deçà ou au-delà du pKA de la molécule, à au moins 2 unités de pH.

**Claims**

1. Process for evaluating the state of the surface of keratinous fibres, especially of human hair and body hair, characterized in that :

a) a specimen of keratinous fibres is immersed in an aqueous immersion solution containing a compound belonging to the benzene or naphthalene series, which has a maximum dimension of $10^{-3}$ µm, which is sufficiently soluble in an aqueous medium to permit the preparation of a solution containing at least 0.01 mole per litre, which is capable of forming, in an aqueous medium, a solution capable of absorbing radiations in the visible and/or the ultraviolet, naturally or after colorimetric development, and whose molar absorbance, measured at the wavelength of the maximum of the absorption spectrum of the said coloured solution, is at least equal to 1 500, the aqueous immersion solution having a pH such that no ionic charge appears on the compound so as to produce an « uptake » of the compound on the said fibres which is a function of the state of the surface of the latter ;

b) the specimen of keratinous fibres is with-drawn from the said solution and the excess of the said solution saturating the fibres is removed by quick washing or dewatering ;

c) the compound is extracted from the fibres of the said specimen by immersing the fibres in at least one aqueous bath whose pH is substantially identical with that of the aqueous immersion solution, to produce an extraction solution which is correspondingly coloured or capable of being coloured by a development ;

d) the colour of the extraction solution is developed, if necessary ; and

e) the state of the surface of the said keratinous fibres is deduced from the quantity of compounds present in the said extraction solution.

2. Process according to Claim 1, characterized in that there is chosen, as a compound which does not require development, a compound of formula :

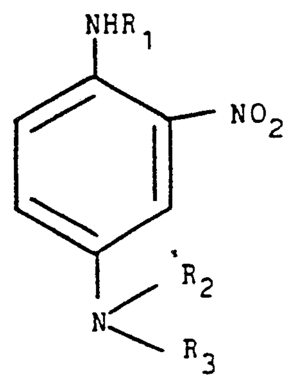

(I)

in which formula :

— $R_1$ denotes H, $CH_2CH_2OH$, $CH_2CH_2NH_2$, $CH_3$ ; and

— $R_2$ and $R_3$ denote, independently, H and $CH_2CH_2OH$.

3. Process according to Claim 1, characterized in that there is chosen, as a compound requiring a development, a compound taken from the group consisting of 1,3-dihydroxynaphthalene, α-naphthol, 2,6-dimethylphenol and 2,4-diaminoanisole sulphate.

4. Process according to one of Claims 1 to 3, characterized in that in step a) an aqueous solution of the chosen compound is employed, which contains a third solvent such as ethanol, the weight ratio (aqueous part/third solvent) being at least equal to 80/20, and especially of the order of 90/10.

5. Process according to one of Claims 1 to 4, characterized in that in step a), an aqueous solution of the chosen compound is employed, in which solution the aqueous part consists of a buffer solution.

6. Process according to Claim 5, characterized in that an aqueous solution whose pH is at least 2 pH units below or above the pKA of the compound is employed.

7. Process according to one of Claims 1 to 6, characterized in that in step a) the immersion of the specimen of keratinous fibres in a proportion of approximately 100 mg of fibres per 2 to 10 ml of solution is produced for at most 20 minutes, preferably for a time of between 2 and 5 minutes, and at a temperature below 50 °C, preferably at a temperature close to ambient temperature.

8. Process according to Claim 1 and 5 taken simultaneously, characterized in that for the extraction in step c) use is made of at least one bath of the same buffer solution as that which has been used to prepare the immersion solution, or of at least one bath of water brought to the same pH as that of the buffer solution which has been used to prepare the immersion solution.

9. Process according to one of Claims 1 to 8, characterized in that the extraction in step c) is carried out for a time of at least 3 minutes.

10. Process according to one of Claims 1 to 9, characterized in that in step e) the extraction solution which is coloured or after development of its colour is compared with a range of dilution of a concentrated solution of the compound in the same medium as that which has been used for the

extraction, or the extraction solution which is coloured or after development of its colour is analysed by means of a spectrophotometer or a colorimeter set to the wavelength of the maximum of the absorption spectrum of the extraction solution which is correspondingly coloured or after development of its colour.

11. Process according to one of Claims 1 to 10, characterized in that in step g) the results are expressed on a colorimetric scale, on a compound concentration scale or on an empirical scale after calibration.

12. Composition for making use of the process according to one of Claims 1 to 11, characterized in that it consists of an aqueous buffer solution containing a compound belonging to the benzene or naphthalene series, which has a maximum dimension of $10^{-3}$ μm, which is sufficiently soluble in an aqueous medium to permit the preparation of a solution containing at least 0.01 mole per litre, which is capable of forming, in an aqueous medium, a solution capable of absorbing radiations in the visible and/or the ultraviolet, naturally or after colorimetric development, and whose molar absorbance, measured at the wavelength of the maximum of the absorption spectrum of the said coloured solution, is at least equal to 1 500, the aqueous buffer immersion solution having a pH such that no ionic charge appears on the compound.

13. Composition according to Claim 12, characterized in that the compounds which do not require development are chosen from those denoted by the formula :

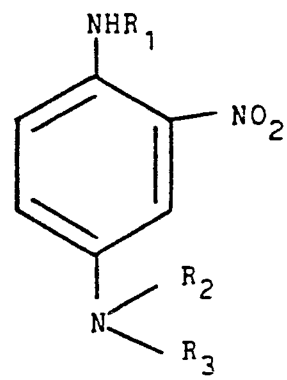

(I)

in which formula :

— $R_1$ denotes H, $CH_2CH_2OH$, $CH_2CH_2NH_2$, $CH_3$ ; and

— $R_2$ and $R_3$ denote, independently, H and $CH_2CH_2OH$.

14. Composition according to Claim 12, characterized in that the compounds requiring a development are taken from the group consisting of 1,3-dihydroxynaphthalene, α-naphthol, 2,6-dimethylphenol and 2,4-diaminoanisole sulphate.

15. Composition according to one of Claims 12 to 14, characterized in that it contains a third solvent such as ethanol, the weight ratio aqueous part/third solvent being at least equal to 80/20, and especially of the order of 90/10.

16. Composition according to one of Claims 12 to 15, characterized in that the aqueous part of the solution consists of a buffer solution having a pH which is at least 2 pH units below or above the pKA of the compound.

**Patentansprüche**

1. Verfahren zur Bestimmung des Zustandes der Oberfläche von Keratinfasern, insbesondere von menschlichen Haaren oder Haaren von Tieren, dadurch gekennzeichnet, dass man

a) eine Probe der Keratinfasern in eine wässerige Tauchlösung, welche ein Molekül, das der Benzol- oder Naphthalinreihe angehört, enthält, das eine maximale räumliche Hinderung von $10^{-3}$ μm besitzt, das in wässerigem Milieu ausreichend löslich ist, um die Herstellung einer Lösung zu ermöglichen, die mindestens 0,01 Mol/l enthält und das geeignet ist, in wässerigem Milieu eine Lösung zu bilden, die fähig ist, von selbst oder nach kolorimetrischer Entwicklung im Sichtbaren und/oder im Ultraviolett Strahlungen zu absorbieren und bei welcher der molare Extinktionskoeffizient, gemessen bei der maximalen Wellenlänge des Absorptionsspektrums der genannten gefärbten Lösung, mindestens gleich 1 500 ist, eintaucht, wobei die wässerige Tauchlösung einen solchen pH-Wert aufweist, bei welchem keine ionische Ladung am Molekül auftritt, um derart ein « Eindringen » des Moleküls in die genannten Fasern, welches eine Funktion des Zustandes der Oberfläche dieser letzteren ist, zu bewirken ;

b) die Probe der Keratinfasern aus der genannten Lösung herauszieht und den Überschuss der die Fasern tränkenden genannten Lösung durch Absaugen oder schnelles Waschen entfernt ;

c) das Molekül aus den Fasern der genannten Probe durch Tauchen der Fasern in mindestens ein wässeriges Bad, dessen pH-Wert genau gleich ist demjenigen der wässerigen Tauchlösung, extrahiert,

um eine Extraktionslösung, welche entweder gefärbt oder durch Entwicklung färbbar ist, zu erhalten ;

d) die Färbung der Extraktionslösung falls erforderlich entwickelt und

e) den Zustand der Oberfläche der genannten Keratinfasern aus der Menge der in der genannten Extraktionslösung enthaltenen Moleküle bestimmt.

2. Verfahren nach Anspruch 1, dadurch gekennzeichnet, dass man als Molekül, welches keine Entwicklung erfordert, ein Molekül der allgemeinen Formel

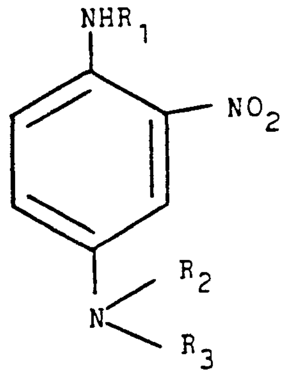

(I)

worin

$R_1$ H, $CH_2CH_2OH$, $CH_2CH_2NH_2$, $CH_3$ und

$R_2$ und $R_3$ unabhängig H und $CH_2CH_2OH$ bedeuten, auswählt.

3. Verfahren nach Anspruch 1, dadurch gekennzeichnet, dass man als Molekül, welches eine Entwicklung erfordert, ein Molekül auswählt aus der Gruppe : 1,3-Dihydroxynaphthalin α-Naphthol, 2,6-Dimethylphenol und 2,4-Diaminoanisolsulfat.

4. Verfahren nach einem der Ansprüche 1 bis 3, dadurch gekennzeichnet, dass man bei dem Verfahrensschritt a) eine wässerige Lösung des ausgewählten Moleküls verwendet, die ein drittes Lösungsmittel, wie Äthanol, enthält, wobei das Gewichtsverhältnis (wässeriger Anteil/drittes Lösungsmittel) mindestens gleich 80/20 ist und insbesondere in der Grössenordnung 90/10 liegt.

5. Verfahren nach einem der Ansprüche 1 bis 4, dadurch gekennzeichnet, dass man bei dem Verfahrensschritt a) eine wässerige Lösung des ausgewählten Moleküls verwendet, wobei der wässerige Anteil der Lösung von einer Pufferlösung gebildet ist.

6. Verfahren nach Anspruch 5, dadurch gekennzeichnet, dass man eine wässerige Lösung verwendet, deren pH-Wert um mindestens 2 pH-Wert-Einheiten oberhalb oder unterhalb des pKA-Wertes des Moleküls liegt.

7. Verfahren nach einem der Ansprüche 1 bis 6, dadurch gekennzeichnet, dass man bei dem Verfahrensschritt a) das Eintauchen der Probe der Keratinfasern in der Weise durchführt, dass ungefähr 100 mg Fasern für 2 bis 10 ml Lösung, während höchstens 20 min, vorzugsweise während eines Zeitraumes zwischen 2 und 5 min und bei einer Temperatur unterhalb 50 °C, vorzugsweise bei einer Temperatur nahe Raumtemperatur, eingetaucht werden.

8. Verfahren nach den Ansprüchen 1 und 5 zusammen, dadurch gekennzeichnet, dass man für die Extraktion des Verfahrensschrittes c) mindestens ein Bad der gleichen Pufferlösung, wie sie bei der Herstellung der Tauchlösung verwendet wurde, oder mindestens ein wässeriges Bad, welches auf den gleichen pH-Wert gebracht wurde, wie derjenige der Pufferlösung, welche zur Herstellung der Tauchlösung verwendet wurde, verwendet.

9. Verfahren nach einem der Ansprüche 1 bis 8, dadurch gekennzeichnet, dass die Extraktion des Verfahrensschrittes c) während eines Zeitraumes von mindestens 3 min durchgeführt wird.

10. Verfahren nach einem der Ansprüche 1 bis 9, dadurch gekennzeichnet, dass man bei dem Verfahrensschritt e) die gefärbte oder die durch Entwicklung gefärbte Extraktionslösung mit einer Verdünnungsreihe ausgehend von einer konzentrierten Lösung des Moleküls in demselben Milieu, welches zur Extraktion verwendet wurde, vergleicht, oder auch die gefärbte oder die durch Entwicklung gefärbte Extraktionslösung mit Hilfe eines Spektrophotometers oder mit einem geeichten Kolorimeter bei der maximalen Wellenlänge des Absorptionsspektrums der gefärbten oder der durch Entwicklung gefärbten Extraktionslösung analysiert.

11. Verfahren nach einem der Ansprüche 1 bis 10, dadurch gekennzeichnet, dass man bei dem Verfahrensschritt e) die Ergebnisse auf einer kolorimetrischen Skala, auf einer Konzentrationsskala des Moleküls oder auf einer willkürlich angenommenen Skala nach Eichung ausdrückt.

12. Mittel zur Durchführung des Verfahrens nach einem der Ansprüche 1 bis 11, dadurch gekennzeichnet, dass es aus einer wässerigen Pufferlösung besteht, die ein Molekül, das der Benzol- oder Naphthalinreihe angehört, enthält, das eine maximale räumliche Hinderung von $10^{-3}$ μm besitzt, das in wässerigem Milieu ausreichend löslich ist, um die Herstellung einer Lösung zu ermöglichen, die mindestens 0,01 Mol/l enthält und das geeignet ist, in wässerigem Milieu eine Lösung zu bilden, die fähig ist, von selbst oder nach kolorimetrischer Entwicklung im Sichtbaren und/oder im Ultraviolett Strahlungen zu absorbieren und bei welchem der molare Extinktionskoeffizient, gemessen bei der maximalen Wellenlänge des Absorptionsspektrums der genannten gefärbten Lösung, mindestens gleich 1 500 ist, wobei die wässerige Tauch-Pufferlösung einen solchen pH-Wert aufweist, bei welchen keine ionische Ladung am Molekül auftritt.

13. Mittel nach Anspruch 12, dadurch gekennzeichnet, dass die Moleküle, welche keine Entwicklung erfordern unter solchen der allgemeinen Formel

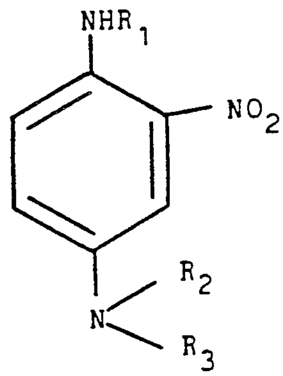

(I)

worin

$R_1$ H, $CH_2CH_2OH$, $CH_2CH_2NH_2$, $CH_3$ und

$R_2$ und $R_3$ unabhängig H und $CH_2CH_2OH$ bedeuten, ausgewählt sind.

14. Mittel nach Anspruch 12, dadurch gekennzeichnet, dass die Moleküle, welche eine Entwicklung erfordern, ausgewählt sind aus der Gruppe : 1,3-Dihydroxynaphthalin, α-Naphthol, 2,6-Dimethylphenol und 2,4-Diaminoanisolsulfat.

15. Mittel nach einem der Ansprüche 12 bis 14, dadurch gekennzeichnet, dass es ein drittes Lösungsmittel, wie Äthanol, enthält, wobei das Gewichtsverhältnis wässeriger Anteil/drittes Lösungsmittel mindestens gleich 80/20 ist und insbesondere in der Grössenordnung 90/10 liegt.

16. Mittel nach einem der Ansprüche 12 bis 15, dadurch gekennzeichnet, dass der wässerige Anteil der Lösung von einer Pufferlösung gebildet ist, welche einen pH-Wert aufweist, der um mindestens 2 pH-Wert-Einheiten unterhalb oder oberhalb des pKA Wertes des Moleküls liegt.